(19) Europäisches Patentamt
European Patent Office
Office européen des brevets

(11) Publication number: **0 014 562**
**B1**

# (12) EUROPEAN PATENT SPECIFICATION

(45) Date of publication of patent specification: **05.10.83**

(21) Application number: **80300275.7**

(22) Date of filing: **30.01.80**

(51) Int. Cl.³: **C 07 D 237/18, C 07 F 3/06, A 61 K 31/50 //C07D237/14**

(54) **Pyridazines for use in treating fungal infections, pharmaceutical compositions containing them, and processes for their preparation.**

(30) Priority: **31.01.79 GB 7903421**

(43) Date of publication of application:
**20.08.80 Bulletin 80/17**

(45) Publication of the grant of the patent:
**05.10.83 Bulletin 83/40**

(84) Designated Contracting States:
**BE CH DE FR GB IT LU NL SE**

(56) References cited:
**GB - A - 2 025 416**

**TETRAHEDRON LETTERS, no. 9, 1962, pages 393—396 Oxford, G.B.**
**R. N. CASTLE et al.: "The nucleophilic displacement of halogen in pyridazines with phosphorous pentasulfide"**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LU NL SE**

(72) Inventor: **Gymer, Geoffrey Edward**
**19 Palmer Road**
**Wingham Canterbury, Kent (GB)**
Inventor: **Richardson, Kenneth**
**48 St. Stephens Hill**
**Canterbury Kent (GB)**

(74) Representative: **Wood, David John et al,**
**Pfizer Limited Ramsgate Road**
**Sandwich Kent CT13 9NJ (GB)**

**EP 0 014 562 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

(56) References cited:

JOURNAL OF THE CHEMICAL SOCIETY, Perkin Transactions I, 1977, pages 1038—1044 London, G.B.
G. B. BARLIN et al.: "Tautomerism in N-Heterocycles. Part 3. 3,4,5-Trimercaptopyridazine"

CHEMICAL ABSTRACTS, vol. 83, no. 8, 25th August 1975, page 846, no. 70824j Columbus, Ohio, U.S.A.
J. S. DWIVEDI et al.: "3,4,5-Pyridazine trithiol as a complexing agent. Complexes of gold(III), lead(II), cadmium(II), silver(I), thallium(I), and mercury(II)"

CHEMICAL ABSTRACTS, vol. 79, no. 16, 22nd October 1973, page 555, no. 99984q Columbus, Ohio, U.S.A.
J. S. DWIVEDI et al.: "3,4,5-Pyridazinetrithiol complexes of copper(II), nickel(II), cobalt(II), iron(III), and chromium(III)"

Pyridazines for use in treating fungal infections, pharmaceutical compositions containing them, and processes for their preparation

This invention relates to certain dimercaptopyridazine thiones, and to their zinc complexes and alkali metal salts, which possess antifungal activity.

According to the invention there is provided a compound of the formula:

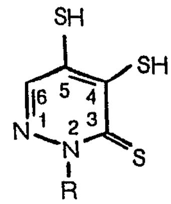

--- (I)

or

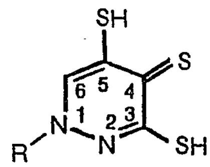

--- (II)

wherein R is H, $C_1$—$C_4$ alkyl, —$CH_2$—$CH_2$ ($C_1$—$C_4$ alkoxy) or benzyl, or a zinc complex thereof, or an alkali metal salt of a compound of the formula (I) or (II) or zinc complex thereof, or a pharmaceutical composition containing said compound of the formula (I) or (II) or zinc complex or salt together with a pharmaceutically acceptable diluent or carrier, for use in treating a fungal infection in a human being.

The compounds of the formulae (I) and (II) in which R is H or $CH_3$ are known, but no medical use has previously been described for them [see J. C. S. Perkin I, 1038 (1977); Chem. Pharm. Bull., 18(5), 970—981 (1970); Tetrahedron Letters No. 9, pp 393—396, 1962; J. Het. Chem., *3*, p. 541 (Dec. 1966); and J. Het. Chem., *3*, p. 79 (March 1966)]. The zinc complexes and the compounds in which R is other than H or $CH_3$ are however novel compounds.

Thus a further aspect of the invention provides a compound of the formula (I) or (II) in which R is $C_1$—$C_4$ alkyl, —$CH_2CH_2$($C_1$—$C_4$ alkoxy) or benzyl, or an alkali metal salt thereof; or a zinc complex of a compound of the formula (I) or (II) in which R is H, $C_1$—$C_4$ alkyl, —$CH_2CH_2$($C_1$—$C_4$ alkoxy) or benzyl, or an alkali metal salt of said complex.

The invention also includes a pharmaceutical composition comprising a compound of the formula (I) or (II) in which R is $C_2$—$C_4$ alkyl, —$CH_2CH_2$($C_1$—$C_4$) alkoxy) or benzyl, or an alkali metal salt thereof; or a zinc complex of a compound of the formula (I) or (II) in which R is H, $C_1$—$C_4$ alkyl, —$CH_2CH_2$($C_1$—$C_4$ alkoxy) or benzyl, or an alkali metal salt of said complex; together with a pharmaceutically acceptable diluent or carrier. The most active compounds are those in which R is H or $CH_3$ and their alkali metal salts and zinc complexes.

The compounds of the formulae (I) and (II) can exist in tautomeric forms, and such tautomers and compositions containing them, are also within the scope of the invention. For example, when R is H, formulae (I) and (II) are tautomeric structures.

The compounds of the formulae (I) and (II) in which R is H or $CH_3$ are, as stated previously, known compounds.

The novel compounds of the invention may be prepared by the following routes:

(i)

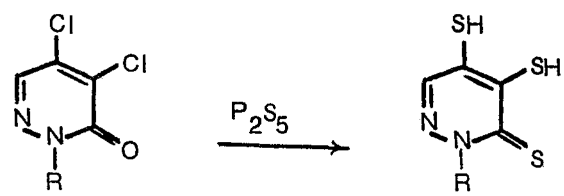


(III) (I)

(R = $C_2$—$C_4$ Alkyl or benzyl).

In a typical procedure, the compound (III) and phosphorous pentasulfide are refluxed together in a suitable solvent, e.g. dry pyridine, for a considerable period of time, e.g. 60—160 hours. The product (I)

can then be obtained by evaporating the solvent under reduced pressure, and heating the resulting residue with water on a steam bath for a few hours. The resulting solution can then be filtered, acidified, e.g. to pH 1 with concentrated hydrochloric acid, and extracted with a suitable solvent, e.g. ethyl acetate. The organic phase can then be dried, and either evaporated to leave an oil which can be crystallized to the desired product, or evaporated to partial volume and cooled to give the product.

The starting materials of the formula (III) are either known compounds or may be prepared by procedures analogous to those of the prior art [see e.g. Angew. Chemie I.E., *4*, 292 (1965) and Chem. Pharm. Bull. (Tokyo), *18*, 154 (1970)].

(2)

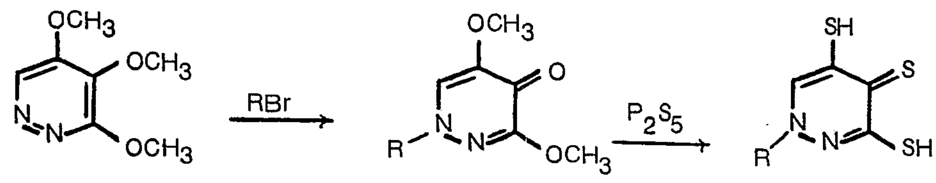


(IV) (V) (II)

(R = Benzyl, $C_2$—$C_4$ alkyl or (($C_1$—$C_4$ alkoxy)$CH_2CH_2$—).

In a typical procedure the starting material (IV) is heated under reflux for a few hours in a suitable solvent with the appropriate bromide. The solution can then be cooled, and the intermediate (V) filtered off, washed with e.g. ether, dried, and recrystallised from a suitable solvent, e.g. isopropanol. The intermediate (V) can then be converted to the end product (II) typically by refluxing with phosphorus pentachloride in dry pyridine.

The starting material (IV) can be prepared as in J. C. S. Perkin 1, 1043, (1977).

(3)

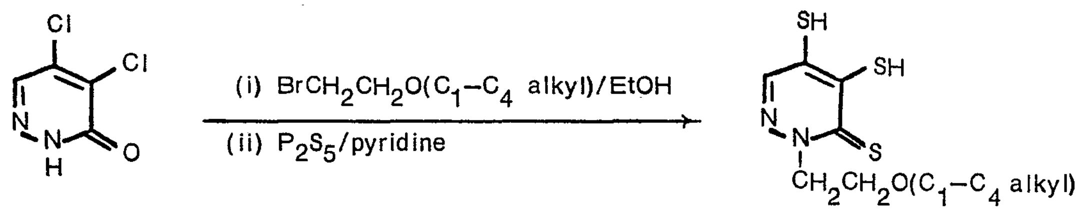


(VI) (I)

In a typical procedure, compound (VI) and the bromide ($C_1$—$C_4$ alkyl)$OCH_2CH_2Br$ are heated together under reflux in the presence of a strong base such as anhydrous potassium carbonate in an alcoholic solvent, preferably ethanol, for several days. The resulting mixture can then be cooled, filtered, the solvent removed under reduced pressure, the residue extracted with a suitable solvent, e.g. ethyl acetate, and the organic phase separated, dried and evaporated to an intermediate product. This intermediate can typically be converted to the desired product (I) by refluxing with phosphorus pentasulfide in dry pyridine.

The starting material (VI) may be prepared as described in J.A.C.S., *75*, 1909 (1953).

(4) The zinc complexes can be prepared by heating, e.g. under reflux, the compound of the formula (I) or (II) with zinc dust and acetic acid in a suitable solvent, e.g. methanol. Heating for about 2 hours is usually sufficient. The complex may be recovered by conventional techniques, e.g. either by cooling or evaporating the reaction mixture to leave a residue, treating this with sodium hydroxide, filtering, and acidifying to e.g. pH 1 with concentrated hydrochloric acid to precipitate the desired zinc complex.

The zinc complexes may also be prepared by reacting the compound of the formula (I) or (II) with zinc acetate, typically in the presence of a base such as sodium hydroxide.

The *in vitro* evaluation of the anti-fungal activity of the compounds of the formulae (I) and (II) [including their zinc complexes] can be performed by determining the minimum inhibitory concentration (m.i.c.) of the test compounds in a suitable medium at which growth of the particular micro-organism fails to occur.

In practice a series of agar plates, each having the test compound incorporated at a particular concentration, are inoculated with a standard culture of *Candida albicans* and each plate is then incubated for 24 hours at 37°C. The plates are then examined for the presence or absence of growth of the fungus and the appropriate m.i.c. value is noted. Other micro-organisms used in such tests include *Cryptococcus neoformans, Aspergillus fumigatus, Trichophyton rubrum, Epidermophyton floccosum, Blastomyces dermatitidis,* and *Torulopsis glabrata.*

The *in vivo* evaluation of the compounds may also be carried out at a series of dose levels by intraperitoneal or intravenous injection or by oral administration, to mice which are inoculated with a

strain of *Candida albicans*. Activity is based on the survival of a treated group of mice after the death of an untreated group of mice following 48 hours observation. The dose level at which the compound provides 50% protection against the lethal effect of the infection may be calculated.

For human use, the anti-fungal compounds, salts and complexes of the invention can be administered alone, but will generally be administered in admixture with a pharmaceutical carrier selected with regard to the intended route of administration and standard pharmaceutical practice.

For example, they may be administered orally in the form of tablets containing such excipients as starch or lactose, or in capsules either alone or in admixture with excipients, or in the form of elixirs or suspensions containing flavouring or colouring agents, or in the form of suppositories or pessaries. The tablets are typically prepared by granulating the ingredients together and compressing the resulting mixture to tablets of the desired size. The capsules are typically prepared by granulating the ingredients together and filling them into hard gelatine capsules of the appropriate size to contain the ingredients. Similarly, the suppositories or pessaries may be prepared by conventional methods.

They may also be injected parenterally, for example, intravenously, intramuscularly or subcutaneously. For parenteral administration, they are best used in the form of a sterile aqueous solution which may contain other solutes, for example, enough salts or glucose to make the solution isotonic. Such formulations may again be prepared by conventional techniques.

For oral and parenteral administration to human patients, it is expected that the daily dosage level of the anti-fungal compounds of the formula (I) or (II) or their complexes will be from 0.5 to 20 mg/kg (in divided doses), when administered by the oral or parenteral route. Thus tablets, capsules or dosage units for parenteral administration (e.g. ampoules) containing the compounds can be expected to contain from 30 mg to 0.5 g of active compound. The physician in any event will determine the actual dosage which will be most suitable for an individual patient and it will vary with the age, weight and response of the particular patient. The above dosages are exemplary of the average host. There can, of course, be individual cases where higher or lower dosage ranges are merited, and such are within the scope of this invention.

Alternatively, the anti-fungal compounds, salts and complexes of the invention may be applied topically, e.g. in the form of a cream or ointment. For example they may be incorporated into a cream consisting of an aqueous emulsion of polyethylene glycol or liquid paraffin; or they may be incorporated, at a concentration between 1 and 10%, into an ointment consisting of a white wax or white soft paraffin base together with such stabilisers and preservatives as may be required.

The following Examples illustrate the invention. All temperatures are given in °C.

Example 1

4,5-Dimercapto-2-ethylpyridazin-3-thione

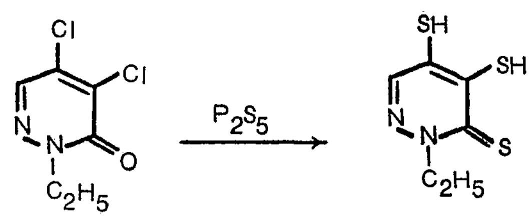


4,5-Dichloro-2-ethylpyridazin-3-one was prepared as described in the literature (Angew. Chemie I.E., *4*, 292 (1965).

This compound (0.7 g), was heated under reflux in pyridine (110 ml) with phosphorus pentasulphide (8 g) for 70 hours.

The pyridine was evaporated under reduced pressure and the residue treated with water (50 ml) and heated on a steam bath for 2 hours. The resulting solution was filtered, acidified to pH 1 with concentrated hydrochloric acid, and extracted with ethyl acetate (3 x 100 ml).

The organic extract was washed with water (1 x 100 ml), dried over magnesium sulphate, filtered and evaporated to a dark oil. This solidified on standing to give the product as a low melting crystalline solid, 0.6 g, m.p. 43—4°C.

The structure was confirmed by mass spectra, n.m.r. and t.l.c.:

*Mass Spectra:* $M^{\oplus}$ 204

*N.M.R.* ($CDCl_3$, 60 MHz): 1.58 $\delta$ triplet, J 7 cps, 3 protons ($CH_3$)
4.75 $\delta$ quartet, J 7 cps, 2 protons ($CH_2$)
7.9 $\delta$ singlet, 1 proton (ring H)

Example 2

Zinc Complex of 4,5-Dimercapto-2-ethylpyridazin-3-thione

The product of Example 1 (0.15 g) was heated under reflux for two hours in methanol (3 ml) with acetic acid (1.4 ml) and zinc dust (0.11 g).

The solvents were then removed under reduced pressure and the resulting yellow solid stirred in water and basified to pH 14 with concentrated sodium hydroxide solution. The mixture was filtered and the filtrate acidified to pH 1 with concentrated hydrochloric acid to give the product as a yellow solid, 120 mg, m.p. 120—128°C.

*Analysis %:*

| | |
|---|---|
| Found: | C, 28.6; H, 2.6; N, 10.9 |
| $C_{24}H_{24}N_8S_{12}.3Zn$ requires: | C, 28.7; H, 2.4; N, 11.15. |

Example 3

2-Benzyl-4,5-Dimercaptopyridazin-3-thione

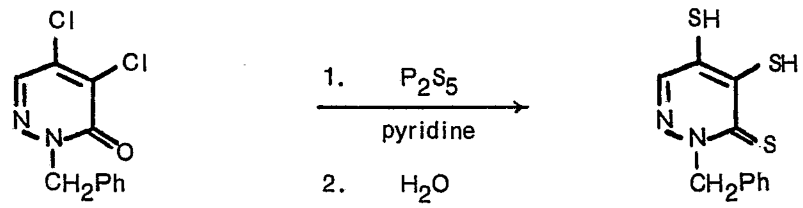


The starting material, 2-benzyl-4,5-dichloropyridazin-3-one, was prepared by the literature route (Chem. Pharm. Bull. (Tokyo) *18*, 154 (1970)).

This material (12.0 g) and phosphorus pentasulphide (106 g, 10 equivalent) were refluxed in dry pyridine (1.2 litres) for 144 hours.

The pyridine was then removed under reduced pressure, the residue heated on a steam bath for 2 hours with water (250 ml). The solution thus obtained was cooled, and acidified to pH 1 with concentrated hydrochloric acid. The aqueous phase was decanted from a gummy precipitate and extracted with ethyl acetate (2 x 400 ml). The precipitate was also extracted with warm ethyl acetate (700 ml) and the organic extracts combined, dried ($MgSO_4$) and evaporated to approximately 75 ml volume. On cooling in ice, the product was deposited as a pale brown solid, which was filtered off and washed with a little dry ether, 6.2 g, m.p. 118—121°.

*Analysis %:*

| | |
|---|---|
| Found: | C, 49.76; H, 3.72; N, 10.71; S, 35.84 |
| $C_{11}H_{10}N_2S_3$ requires: | C, 49.63; H, 3.79; N, 10.52; S, 36.06. |

Example 4

Zinc Complex of 2-Benzyl-4,5-Dimercaptopyridazin-3-thione

2-Benzyl-4,5-dimercaptopyridazin-3-thione (2.5 g), zinc dust (1.35 g), and acetic acid (17 ml) were heated under reflux in methanol (35 ml) for 2 hours.

The mixture was then cooled in ice and the insolubles filtered off and dissolved in aqueous sodium hydroxide.

The sodium hydroxide solution was filtered and acidified to pH 1 with concentrated hydrochloric acid. The precipitate was filtered off and dried (0.9 g). Recrystallisation from aqueous dimethylformamide gave the product as a yellow solid, 0.5 g, m.p. 210—230°.

*Analysis %:*

| | |
|---|---|
| Found: | C, 44.21; H, 3.00; N, 9.54 |
| $C_{22}H_{18}N_4S_6.Zn$ requires: | C, 44.35; H, 3.05; N, 9.40 |

Example 5

1-Benzyl-3,5-Dimercaptopyridazin-4-thione

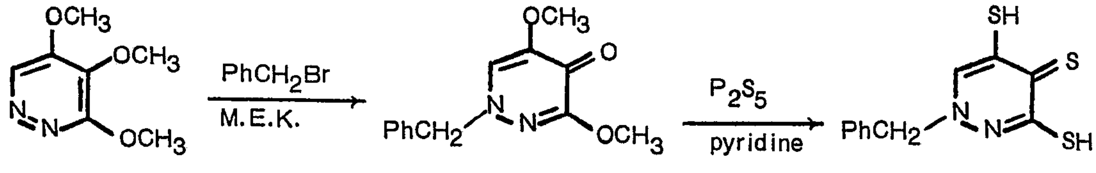


A. *1-Benzyl-3,5-dimethoxypyridazin-4-one*

The starting material 3,4,5-trimethoxypyridazine, was prepared by the literature route (J. C. S. Perkin 1, 1043 (1977)).

This material (5.5 g) was heated under reflux for two hours, with benzyl bromide (5.5 g) in methyl ethyl ketone (30 ml). On cooling in ice a crystalline precipitate formed, which was filtered off, washed with ether and dried (5 g).

Recrystallisation from isopropyl alcohol gave the product as a crystalline solid (4.0 g), m.p. 147—149°C.

*Analysis %:*

| | |
|---|---|
| Found: | C, 64.18; H, 5.58; N, 10.76 |
| $C_{13}H_{14}N_2O_3$ requires: | C, 63.40; H, 5.73; N, 11.38. |

B. *1-Benzyl-3,5-Dimercaptopyridazin-4-thione*

1-Benzyl-3,5-dimethoxypyridazin-4-one (4 g) and phosphorus pentasulphide (22 g) were heated under reflux in dry pyridine (300 ml) for 2.5 hours. The pyridine was then removed under reduced pressure and the residue heated on a steam bath for two hours with water (60 ml).

The solution thus obtained was cooled and acidified to pH 1 with concentrated hydrochloric acid. A black tarry precipitate formed which was stirred in chloroform (250 ml), when most of it dissolved. The aqueous mother liquors were extracted with chloroform (2 x 250 ml) and the combined chloroform extracts were washed with water, dried ($MgSO_4$) and evaporated to a mobile red oil.

This oil was taken up in sodium hydroxide solution (50 ml., 10N) and the solution filtered through a bed of "Hyflo" (Trade Mark). On acidification to pH 1 with concentrated hydrochloric acid a gum was deposited which solidified on trituration. This solid was filtered off, washed with water and dried at 50° under vacuum.

This solid was stirred with dry ether (10 ml) to give the product as a yellow crystalline solid, 2.0 g, m.p. 85—108°.

*Analysis %:*

| | |
|---|---|
| Found: | C, 49.63; H, 3.84; N, 10.32 |
| $C_{11}H_{10}N_2S_3$ requires: | C, 49.63; H, 3.79; N, 10.52. |

Example 6

Zinc Complex of 1-Benzyl-3,5-Dimercaptopyridazin-4-thione

1-Benzyl-3,5-dimercaptopyridazin-4-thione (0.5 g), zinc dust (0.3 g) and acetic acid (4 ml) were heated under reflux with stirring in methanol (8 ml) for 2 hours. The mixture was then cooled, the solid filtered off, dissolved in sodium hydroxide solution (4 ml, 10N), the solution filtered and acidified to pH 1 with concentrated hydrochloric acid to give the product as a yellow solid, m.p. 180—188° (decomp.), 0.23 g.

*Analysis %:*

| | |
|---|---|
| Found: | C, 41.18; H, 2.67; N, 9.21 |
| $C_{44}H_{34}N_8S_{12}.3Zn$ requires: | C, 42.1; H, 2.71; N, 8.94. |

Example 7

4,5-Dimercapto-2-(2-ethoxyethyl)pyridazin-3-thione

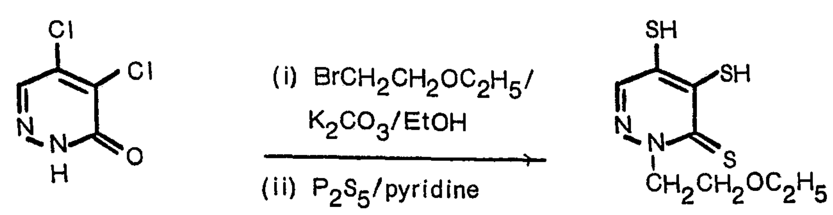


4,5-Dichloropyridazine-3-one was prepared by the literature method (J.A.C.S. *75*, 1909 (1953)).

A. *4(5)-Chloro-5(4)-ethoxy-2-(2-ethoxyethyl)pyridazin-3-one*

4,5-Dichloropyridazin-3-one (5 g), 2-bromoethyl ethyl ether (4.64 g) and anhydrous potassium carbonate (12.5 g) were stirred and heated under reflux in absolute ethanol (250 ml) for 70 hours.

The mixture was then cooled, the inorganic salts filtered off, and the ethanol removed under reduced pressure. The residue was partitioned between ethyl acetate (100 ml) and water (50 ml), the organic layer separated, dried ($MgSO_4$) and evaporated to an orange oil (4.5 g), which eventually crystallised on standing under vacuum for one week.

This material was characterised as being either 4-chloro-5-ethoxy or 5-chloro-4-ethoxy-2-(2-ethoxyethylpyridazin-3-one by mass spectrometry, i.r. and n.m.r.

B. *4,5-Dimercapto-2-(2-ethoxyethyl)pyridazin-3-thione*

4(5)-Chloro-5(4)-ethoxy-2-(2-ethoxyethyl)pyridazin-3-one (1.2 g) and phosphorus pentasulphide (8.0 g) were heated under reflux in dry pyridine (65 ml) for 40 hours.

The pyridine was removed under reduced pressure and the residue heated with water (20 ml) on a steam bath for 2 hours. The solution was then cooled, acidified to pH 1 with concentrated hydrochloric acid and extracted with chloroform. The chloroform layer was washed with water, dried ($MgSO_4$) and evaporated to a red oil.

This material was taken up in dry tetrahydrofuran and the volume reduced by a stream of dry nitrogen until a small quantity of solid was deposited. The solid was filtered off and the filtrate evaporated to a red oil (0.3 g). This oil was characterised by n.m.r. and mass spectrometry as being the desired product.

## Example 8

Zinc Complex of 4,5-Dimercapto-2-(2-ethoxyethyl)pyridazin-3-thione

The product from the preceding Example (0.25 g), zinc dust (0.15 g) and glacial acetic acid (2 ml) were heated under reflux in methanol (4 ml) for two hours.

The solvents were evaporated, the residue stirred in water (3 ml) and basified to pH 14 with sodium hydroxide solution. This mixture was filtered and acidified to pH 1 with concentrated hydrochloric acid, when the desired product was deposited as a pale yellow solid, which was filtered off, washed with ethanol and dried, 0.11 g, m.p. 80—85°.

The structure of the product was confirmed by n.m.r.:

*N.M.R.* ($DMSOd_6$ 60MHz)
1.2 triplet J 7 cps, 3 protons ($CH_3$)
3.5 quartet J 7 cps, 2 protons ($CH_2$ of ethyl)
3.9 multiplet 2 protons ($CH_2$ next to the ring N atom)
4.8 multiplet 2 protons ($CH_2$ next to the O atom)
8.1 singlet 1 proton (ring H)

## Example 9

Zinc Complex of 4,5-Dimercapto-pyridazin-3-thione

To a slurry of 4,5-dimercapto-pyridazin-3-thione (25 g) in water (250 ml) was added 40% sodium hydroxide solution to pH 10. The resulting solution was cooled to 10°C. To this solution was added zinc acetate (15.59 g) in water (250 ml). The resulting mixture was stirred and adjusted to pH 1 with 5N hydrochloric acid. The precipitate was granulated at 5° for 1½ hours, collected, washed and vacuum dried at 50° to give the title zinc complex as an orange/yellow solid (31.2 g), m.p. >300°.

*Analysis %:*

| | |
|---|---|
| Found: | C, 22.07; H, 1.45; N, 12.64 |
| Calculated for $C_8H_6N_4S_6.Zn$: | C, 23.10; H, 1.45; N, 13.47. |

## Example 10

By a similar procedure to Example 9, starting from 2-methyl-4,5-dimercaptopyridazin-3-thione and zinc acetate, the zinc complex of 2-methyl-4,5-dimercaptopyridazin-3-thione was prepared, m.p. > 300°.

*Analysis %:*

| | |
|---|---|
| Found: | C, 26.49; H, 2.21; N, 12.64 |
| Calculated for $C_{10}H_{10}N_4S_6.Zn$: | C, 27.05; H, 2.27; N, 12.62. |

## Example 11

Zinc Complex of 4,5-Dimercapto-pyridazin-3-thione

4,5-Dimercapto-pyridazin-3-thione (20 g) was added to a stirred mixture of powdered zinc (3.71 g), methanol (93 ml) and glacial acetic acid (37 ml).

The mixture was stirred at reflux for 2½ hours, then cooled to 5° and granulated at 5° for ½ hour. The solid was collected and slurried in 2N sodium hydroxide solution (430 ml). The mixture was clarified by filtration and cooled to 10° prior to acidifying with 5N hydrochloric acid to pH 1.

The slurry was granulated at 5° for ½ hour, the solid collected, washed and vacuum dried at 50°C to give the title complex as an orange-brown solid (20.7 g), m.p. >300°. The product was identical to that of Example 9.

## Example 12

By a procedure similar to that of Example 11, starting from powdered zinc and 2-methyl-4,5-dimercaptopyridazin-3-thione, the zinc complex of 2-methyl-4,5-dimercaptopyridazin-3-thione was prepared. The product was identical to that of Example 10.

Example 13

The following are typical tablet or capsule formulations containing 4,5-dimercapto-pyridazin-3-thione, 1-methyl-3,5-dimercapto-pyridazin-4-thione or 2-methyl-4,5-dimercaptopyridazin-3-thione:—

| | mg/tablet or capsule | | |
|---|---|---|---|
| | A | B | C |
| Active ingredient | 500 | 200 | 200 |
| Lactose | 30 | 125 | — |
| Maize starch | 60 | 70 | — |
| Microcrystalline cellulose ("Avicel") | — | — | 120 |
| Glycine | — | — | 70 |
| Fine silica ("Aerosil") | — | 0.35 | 0.35 |
| Magnesium stearate* | 5 | 3 | 3 |
| | 595 | 398.35 | 393.35 |

* 9:1 blend with sodium lauryl sulphate
"Avicel" and "Aerosil" are Trade Marks.

For formulations A and B the ingredients are thoroughly blended together, and then either filled directly into hard gelatin capsules of appropriate size, or granulated and compressed into tablets of the desired size. For formulation C, the ingredients are thoroughly blended together and slugged. The slugs are broken down into granules, and then either filled into capsules of the appropriate size, or directly compressed into tablets of the appropriate size.

In formulations A and B, the lactose may be replaced by equal amounts of calcium carbonate or dicalcium phosphate.

Example 14

A number of parenteral formulations were prepared by mixing the ingredients specified below in a conventional manner:—

*Number 1* A solution for injection of 15 mg/ml and made isotonic with blood by sodium chloride

| | | |
|---|---|---|
| 4,5-Dimercapto-pyridazin-3-thione | | 1.50 g |
| Sodium Hydroxide 0.2N | Ph. Eur. | 60.0 ml |
| Sodium Chloride | Ph. Eur. | 0.40 g |
| Water for Injection | Ph. Eur. to | 100.0 ml |

*Number 2* A solution for injection of 15 mg/ml and made isotonic with blood by dextrose

As No. 1 above except the sodium chloride was replaced by:

| | | |
|---|---|---|
| Dextrose (anhydrous) | Ph. Eur. | 3.20 g |

*Number 3* A solution for injection of 15 mg/ml at pH 9 and made isotonic with blood by sodium chloride.

| | | |
|---|---|---|
| 4,5-Dimercapto-pyridazin-3-thione | | 1.50 g |
| Sodium Hydroxide 0.2N | Ph. Eur. | 60.0 ml |
| Sodium Chloride | Ph. Eur. | 0.40 g |
| Hydrochloric Acid 1.0N | Ph. Eur. to pH | 9.0 ± 0.1 |
| Water for Injection | Ph. Eur. to | 100.0 ml |

*Number 4* A solution for injection of 15 mg/ml at pH 9 and made isotonic with blood by dextrose.
As No. 3 above except that the sodium chloride was replaced by:

| | | |
|---|---|---|
| Dextrose (anhydrous) | Ph. Eur. | 3.20 g |

*Number 5* An infusion concentrate of 15 mg/ml suitable for dilution with isotonic saline or dextrose
As No. 1 except no sodium chloride was added.

*Number 6* An infusion concentrate of 15 mg/ml at pH 9 suitable for dilution with isotonic saline or dextrose
As No. 3 except that no sodium chloride was added.

*Number 7* An infusion fluid made isotonic with blood by sodium chloride and containing 0.3 mg/ml of active ingredient

| | | |
|---|---|---|
| 4,5-Dimercapto-pyridazin-3-thione | | 60.0 mg |
| Sodium Hydroxide 0.2N | Ph. Eur. | 2.4 ml |
| Sodium Chloride | Ph. Eur. | 1.8 g |
| Water for Injection | Ph. Eur. to | 200.0 ml |

*Number 8* An infusion fluid made isotonic with blood by dextrose and containing 0.3 mg/ml of active ingredient
As No. 7 except that sodium chloride was replaced by:

| | | |
|---|---|---|
| Dextrose (anhydrous) | Ph. Eur. | 10.0 g |

*Number 9* An infusion fluid made isotonic with blood by sodium chloride and containing 0.3 mg/ml of active ingredient with the pH adjusted to pH 9

| | | |
|---|---|---|
| 4,5-Dimercapto-pyridazin-3-thione | | 60.0 mg |
| Sodium Hydroxide 0.2N | Ph. Eur. | 2.4 ml |
| Sodium Chloride | Ph. Eur. | 1.8 g |
| Hydrochloric Acid 1.0N | Ph. Eur. to pH | 9.0 ± 0.1 |
| Water for Injection | Ph. Eur. to | 200.0 ml |

*Number 10* An infusion fluid made isotonic with blood by dextrose and containing 0.3 mg/ml of active ingredient with the pH adjusted to pH 9
As No. 9 except that sodium chloride was replaced by:

| | | |
|---|---|---|
| Dextrose (anhydrous) | Ph. Eur. | 10.0 g |

The final solutions were sterilized in each case according to the method of the BP 1973 by filtration through a suitable bacteria-proof filter under aseptic conditions into sterile containers to comply with the test for sterility of the BP 1973 (Appendix 121). Suitable containers for injectable solutions are small glass vials filled to contain about 4 ml of the solution, and for infusion fluids glass bottles filled to contain 200 ml of the infusion solution. Similarly the infusion concentrates are filled into glass bottles.

**Claims**

1. A compound of the formula:

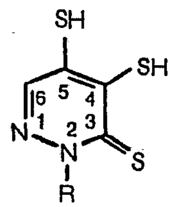

--- (I)

*or*

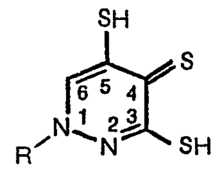

--- (II)

wherein R is H, $C_1$—$C_4$ alkyl, —$CH_2CH_2$($C_1$—$C_4$ alkoxy) or benzyl; or a zinc complex thereof, or an alkali metal salt of a compound of the formula (I) or (II) or zinc complex thereof for use in treating a fungal infection in a human being.

2. A compound for use in treating a fungal infection as claimed in claim 1 in which R in formulae (I) and (II) is H or $CH_3$.

3. A pharmaceutical composition for use in treating a fungal infection in a human being containing a compound of the formula (I) or (II) or zinc complex or salt as claimed in claim 1 together with a pharmaceutically acceptable diluent or carrier.

4. A pharmaceutical composition according to claim 3 containing a compound of formula (I) or (II) as claimed in claim 2.

5. A compound of the formula:

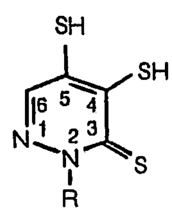

--- (IA)

*or*

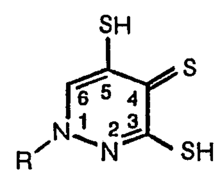

--- (IIA)

wherein R is $C_2$—$C_4$ alkyl, —$CH_2CH_2$($C_1$—$C_4$ alkoxy) or benzyl; or an alkali metal salt thereof.

6. A zinc complex of a compound of the formula (I) or (II) as defined in claim 1, or an alkali metal salt thereof.

7. A pharmaceutical composition comprising a compound of the formula (IA) or (IIA) or alkali metal salt thereof as claimed in claim 5, or a zinc complex or alkali metal salt as claimed in claim 6, together with a pharmaceutically acceptable diluent or carrier.

8. A process for preparing a compound of the formula (IA) as claimed in claim 5 wherein R is $C_2$—$C_4$ alkyl or benzyl, which comprises reacting a compound of the formula:

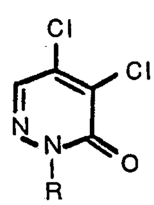

--- (III)

wherein R is as defined above in this method, with phosphorus pentasulphide.

9. A process for preparing a compound of the formula (IIA) as claimed in claim 5, which comprises reacting a compound of the formula:

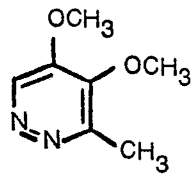

--- (IV)

with a compound of the formula R.Br wherein R is as claimed in claim 5 so as to produce a compound of the formula:

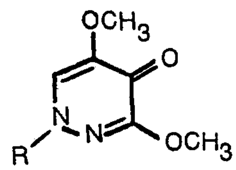

---(V),

followed by reacting the compound of the formula (V) with phosphorus pentasulphide.

10. A process for preparing a compound of the formula (IA) as claimed in claim 5 wherein R is $-CH_2CH_2(C_1-C_4$ alkoxy), which comprises reacting a compound of the formula:

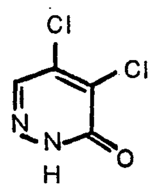

--- (VI),

with a compound of the formula R.Br wherein R is as defined above in this method in the presence of a strong base and in an alcoholic solvent, followed by reacting the resulting product with the phosphorus pentasulphide.

11. A process for preparing a zinc complex as claimed in claim 6, which comprises reacting a compound of the formula (I) or (II) as defined in claim 1 with zinc dust in the presence of acetic acid and/or zinc acetate.

**Patentansprüche**

1. Eine Verbindung der Formel

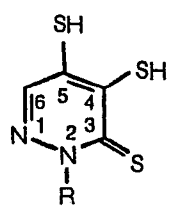

--- (I)

*oder*

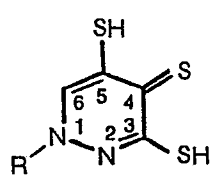

--- (II)

worin R die Bedeutung H, $C_1-C_4$ Alkyl, $-CH_2CH_2(C_1-C_4$ Alkoxy) oder Benzyl besitzt; oder ein Zinkkomplex davon, oder ein Alkalimetallsalz einer Verbindung der Formel (I) oder (II) oder ein Zinkkomplex davon, zur Anwendung bei der Behandlung einer Pilzinfektion in einem menschlichen Wesen.

2. Eine Verbindung zur Anwendung bei der Behandlung einer Pilzinfektion gemäß Anspruch 1 in welcher R in den Formeln (I) und (II) die Bedeutung von H oder $CH_3$ besitzt.

3. Eine pharmazeutische Zusammensetzung zur Anwendung bei der Behandlung einer Pilzinfektion in einem menschlichen Wesen enthaltend eine Verbindung der Formel (I) oder (II) oder einen Zinkkomplex oder ein Salz davon gemäß Anspruch 1 zusammen mit einem pharmazeutisch annehmbaren Verdünnungsmittel oder Träger.

4. Eine pharmazeutische Zusammensetzung gemäß Anspruch 3 enthaltend eine Verbindung der Formel (I) oder (II) gemäß Anspruch 2.

5. Eine Verbindung der Formel

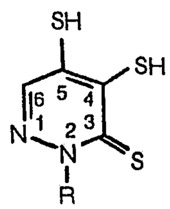

--- (IA)

*oder*

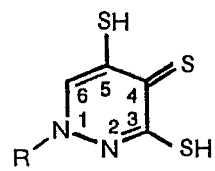

--- (IIA)

worin R die Bedeutung $C_2$—$C_4$ Alkyl, —$CH_2CH_2(C_1$—$C_4$ Alkoxy) oder Benzyl besitzt; oder ein Alkalimetallsalz davon.

6. Ein Zinkkomplex einer Verbindung der Formel (I) oder (II) gemäß Anspruch 1 oder ein Alkalimetallsalz davon.

7. Eine pharmazeutische Zusammensetzung enthaldend eine Verbindung der Formel (IA) oder (IIA) oder ein Alkalimetallsalz davon gemäß Anspruch 5 oder einen Zinkkomplex oder ein Alkalimetallsalz gemäß Anspruch 6 zusammen mit einem pharmazeutisch annehmbaren Verdünnungsmittel oder Träger.

8. Ein Verfahren zur Herstellung einer Verbindung der Formel (IA) gemäß Anspruch 5 worin R $C_2$—$C_4$ Alkyl oder Benzyl bedeutet, wobei eine Verbindung der Formel

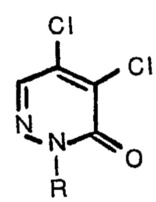

--- (III)

worin R die obige Bedeutung hat mit Phosphorpentasulfid umgesetzt wird.

9. Ein Verfahren zur Herstellung einer Verbindung der Formel (IIA) gemäß Anspruch 5 wobei eine Verbindung der Formel

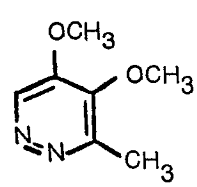

--- (IV)

mit einer Verbindung der Formel R.Br, worin R die in Anspruch 5 gegebene Bedeutung besitzt, umgesetzt wird un eine Verbindung der Formel

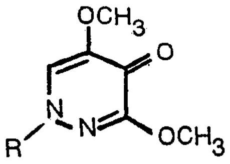

---(V),

herzustellen gefolgt von einer Umsetzung dieser Verbindung der Formel (V) mit Phosphorpentasulfid.

10. Ein Verfahren zur Herstellung einer Verbindung der Formel (IA) gemäß Anspruch 5 worin R die Bedeutung $—CH_2CH_2(C_1—C_4$ Alkoxy) besitzt, wobei eine Verbindung der Formel

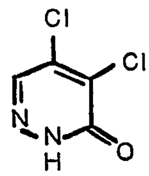

--- (VI),

mit einer Verbindung der Formel R.Br, in welcher R die obige Bedeutung hat, in Anwesenheit einer starken Base in einem alkoholischen Lösungsmittel umgesetzt wird, gefolgt von einer Umsetzung des entstandenen Produktes mit Phosphorpentasulfid.

11. Ein Verfahren zur Herstellung eines Zinkkomplexes gemäß Anspruch 6 wobei eine Verbindung der Formel (I) oder (II) wie in Anspruch 1 definiert mit Zinkstaub in der Anwesenheit von Essigsäure und/oder Zinkacetat umgesetzt wird.

**Revendications**

1. Composé de formule

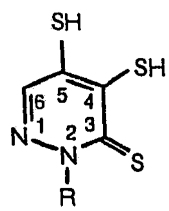

--- (I)

ou

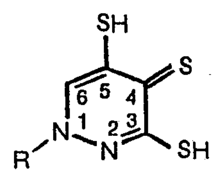

--- (II)

dans laquelle R représente H, un groupe alkyle en $C_1$ à $C_4$; $—CH_2CH_2$(alkoxy en $C_1$ à $C_4$) ou benzyle; ou un complexe de zinc de ce composé, ou un sel de métal alcalin d'un composé de formule (I) ou (II) ou de son complexe de zinc, destiné à être utilisé dans le traitement d'un infection fongique chez un être humain.

2. Composé destiné à être utilisé dans le traitement d'une infection fongique suivant la revendication 1, dans lequel R, dans les formules (I) et (II), représente H ou $CH_3$.

3. Composition pharmaceutique destinée à être utilisée dans le traitement d'une infection fongique chez un être humain, contenant un composé de formule (I) ou (II) ou un complexe de zinc ou un sel suivant la revendication 1 conjointement avec un diluant ou support pharmaceutiquement acceptable.

4. Composition pharmaceutique suivant la revendication 3, contenant un composé de formule (I) ou (II) suivant la revendication 2.

5. Composé de formule:

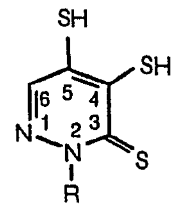

---(IA)

ou

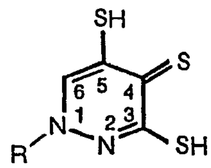

---(IIA)

dans laquelle R est un groupe alkyle en $C_2$ à $C_4$, —$CH_2CH_2$(alkoxy en $C_1$ à $C_4$) ou benzyle; ou un sel de métal alcalin de ce composé.

6. Complexe de zinc d'un composé de formule (I) ou (II) suivant la revendication 1, ou son sel de métal alcalin.

7. Composition pharmaceutique comprenant un composé de formule (IA) ou (IIA) ou un sel de métal alcalin de ce composé suivant la revendication 5, ou un complexe de zinc ou un sel de métal alcalin suivant revendication 6, conjointement avec un diluant ou support pharmaceutiquement acceptable.

8. Procédé de préparation d'un composé de formule (IA) suivant la revendication 5, dans laquelle R est un groupe alkyle en $C_2$ à $C_4$ ou benzyle, qui consiste à faire réagir un composé de formule:

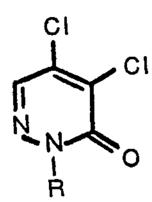

--- (III)

dans laquelle R a la définition donnée ci-dessus dans ce procédé, avec le pentasulfure de phosphore.

9. Procédé de préparation d'un composé de formule (IIA) suivant la revendication 5, qui consiste à faire réagir un composé de formule:

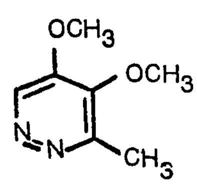

--- (IV)

avec un composé de formule R.Br dans laquelle R a la définition donnée dans la revendication 5 de manière à produire un composé de formule:

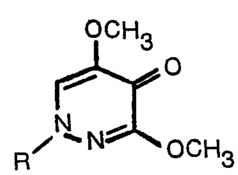

---(V),

puis à faire réagir le composé de formule (V) avec le pentasulfure de phosphore.

10. Procédé de préparation d'un composé de formule (IA) suivant la revendication 5, dans laquelle R est un groupe —$CH_2CH_2$(alkoxy en $C_1$ à $C_4$), qui consiste à faire réagir un composé de formule:

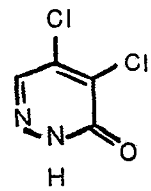

--- (VI),

avec un composé de formule R.Br dans laquelle R a la définition donnée ci-dessus dans ce procédé, en présence d'une base forte et dans un solvant alcoolique, puis à faire réagir le produit résultant avec le pentasulfure de phosphore.

11. Procédé de préparation d'un complexe de zinc suivant la revendication 6, qui consiste à faire réagir un composé de formule (I) ou (II) comme défini dans la revendication 1 avec du zinc en poudre en présence d'acide acétique ou/ou d'acétate de zinc.